(19) Europäisches Patentamt / European Patent Office / Office européen des brevets

(11) **EP 3 651 661 B1**

(12) **FASCICULE DE BREVET EUROPÉEN**

(45) Date de publication et mention de la délivrance du brevet:
**18.08.2021 Bulletin 2021/33**

(21) Numéro de dépôt: **18750241.4**

(22) Date de dépôt: **03.07.2018**

(51) Int Cl.:
**A61B 17/122** *(2006.01)*

(86) Numéro de dépôt international:
**PCT/FR2018/000185**

(87) Numéro de publication internationale:
**WO 2019/012190 (17.01.2019 Gazette 2019/03)**

(54) **ÉLÉMENT POUR RÉALISER UNE PINCE VASCULAIRE ET PINCE VASCULAIRE AINSI RÉALISÉE**

ELEMENT ZUR HERSTELLUNG EINER GEFÄSSKLAMMER UND DAMIT HERGESTELLTE GEFÄSSKLAMMER

ELEMENT FOR PRODUCING A VASCULAR CLIP AND VASCULAR CLIP PRODUCED IN THIS WAY

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **12.07.2017 FR 1770747**

(43) Date de publication de la demande:
**20.05.2020 Bulletin 2020/21**

(73) Titulaire: **Renard, Xavier**
**91430 Vauhallan (FR)**

(72) Inventeurs:
• **RENARD, Jean-Baptiste**
**69002 Lyon (FR)**
• **RENARD, Xavier**
**91430 Vauhallan (FR)**

(74) Mandataire: **Flavenot, Bernard**
**ABRITT**
**17, rue du Docteur Charcot**
**91290 La Norville (FR)**

(56) Documents cités:
**US-A- 3 598 125     US-A- 4 800 879**
**US-A- 5 571 125     US-A1- 2013 289 586**

## Description

La présente invention concerne un perfectionnement aux éléments pour réaliser une pince vasculaire, ainsi que les pinces vasculaires réalisées avec de tels éléments.

Il est déjà connu des éléments pour la réalisation de pinces vasculaires. De tels éléments sont par exemple décrits et illustrés dans les US 5 571 125, EP 0 105 414, US 5 103 839 et WO 2004/073487.

Ces éléments pour réaliser une pince vasculaire donnent relativement satisfaction, mais présente malgré tout des inconvénients, surtout du fait de leurs très petites dimensions.

Aussi, la présente invention propose-t-elle un élément pour réaliser une pince vasculaire, qui pallie en grande partie les inconvénients des éléments similaires de l'art antérieur, ainsi qu'une pince vasculaire réalisée avec de tels éléments, dont la structure permet notamment d'obtenir un prix de revient à la fabrication plus faible tout en conférant à cette pince des caractéristiques de pincement qui sont meilleures que celles des pinces vasculaires de l'art antérieur, bien que présentant un encombrement minimum, ce qui est très important pour les applications de ces pinces, par exemple pour abouter ou maintenir de très petits vaisseaux sanguins comme ceux que l'on trouve dans la main d'un être humain.

Plus précisément, la présente invention a pour objet un élément pour réaliser une pince vasculaire, comportant :

- une plaque de support,
- une patte de pince comportant une face de préhension et définissant un axe longitudinal,
- des moyens pour solidariser ladite patte de pince avec la tranche de ladite plaque,
- deux cavaliers sensiblement identiques, chaque cavalier ayant sensiblement la forme d'un secteur de cylindre d'une épaisseur Ep prise selon une parallèle à la génératrice de définition du secteur de cylindre, et comportant un orifice traversant ayant un diamètre déterminé et d'axe parallèle à ladite génératrice,
- des moyens pour solidariser les deux cavaliers avec une portion de face de ladite plaque de façon que les axes des deux orifices traversants soient situés sur une même droite perpendiculaire à l'axe longitudinal de ladite patte de pince et que les faces en regard des deux cavaliers soient distantes l'une de l'autre d'une valeur égale à l'épaisseur Ep,

caractérisé par le fait que lesdits moyens pour solidariser les deux cavaliers avec la portion de face de ladite plaque sont agencés de façon qu'un des deux cavaliers ait une face située dans le plan d'un bord de la tranche de la plaque perpendiculaire à la droite, et que ladite portion de face soit sensiblement plane, sa largeur prise selon une direction parallèle à ladite droite étant au moins sensiblement égale à quatre fois l'épaisseur Ep, de façon à définir, sur la portion de face, une partie libre bordant la face de l'autre cavalier qui est la plus éloignée de la face du cavalier située dans le plan d'un bord de ladite tranche.

D'autres caractéristiques et avantages de la présente invention apparaîtront au cours de la description suivante donnée en regard des dessins annexés à titre illustratif, mais nullement limitatif, dans lesquels :

La figure 1 est une vue en perspective cavalière d'un premier mode de réalisation d'un élément selon l'invention pour réaliser une pince vasculaire,
La figure 2 est une vue de côté d'une pince vasculaire selon l'invention obtenue avec deux éléments selon l'invention réalisés selon un second mode de réalisation, et
La figure 3 est une vue en coupe transversale de la pince vasculaire, dans le plan *III-III* défini sur la figure 2.

Il est précisé que, dans la présente description, si l'adverbe "sensiblement" est associé à un qualificatif d'un moyen donné, ce qualificatif doit être compris au sens strict ou approché.

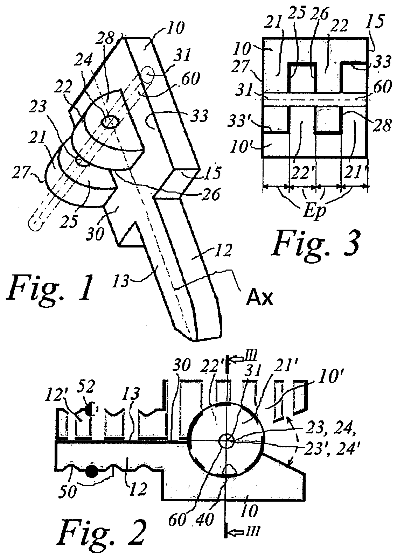

En référence plus particulièrement à la figure 1, la présente invention est relative à un élément pour réaliser une pince vasculaire, cet élément comportant une plaque de support 10 présentant de préférence la forme d'un parallélépipède rectangle, comme illustré sur cette figure 1.

L'élément comporte en outre une patte de pince 12 comportant une face de préhension 13 et définissant un axe longitudinal, des moyens pour solidariser la patte de pince avec la tranche 15 de la plaque 10, deux cavaliers 21, 22 sensiblement identiques, chaque cavalier ayant sensiblement la forme d'un secteur de cylindre, par exemple avantageusement la forme d'un demi-cylindre de révolution.

Chaque cavalier 21, 22 a une épaisseur de valeur Ep, cette dimension étant prise selon une parallèle à la génératrice de définition du secteur de cylindre, et comporte un orifice traversant 23, 24 ayant un diamètre déterminé, l"axe de cet orifice traversant étant parallèle à la génératrice de définition du cylindre.

L'élément comporte en outre des moyens pour solidariser les deux cavaliers 21, 22 avec une portion de face 30 de la plaque 10 de façon que les axes des deux orifices traversants 23, 24 soient situés sur une même droite 31 perpendiculaire à l'axe longitudinal de la patte de pince 12 et que les faces en regard 25, 26 des deux cavaliers 21, 22 soient distantes l'une de l'autre d'une valeur égale à l'épaisseur Ep.

Les moyens pour solidariser la patte de pince 12 avec la tranche 15 de la plaque 10 et les moyens pour solidariser les deux cavaliers 21, 22 avec une portion de face 30 de la plaque 10 peuvent être de tous types. Cependant, selon une réalisation très préférentielle et économiquement avantageuse, ils sont constitués par le fait que la patte de pince 12, les deux cavaliers 21, 22 et la plaque 10 sont réalisés d'une seule pièce par moulage avec une matière plastique ou analogue, la solidarisation de ces quatre pièces entre elles étant de facto obtenue.

Selon une réalisation très préférentielle, les moyens pour solidariser les deux cavaliers 21, 22 avec la portion de face 30 de la plaque 10 sont agencés de façon qu'un 21 des deux cavaliers ait une face 27 située dans le plan d'un bord de la tranche 15 de la plaque 10 perpendiculaire à la droite 31, et que la portion de face 30 soit sensiblement plane, la largeur de cette portion de face 30 prise selon une direction parallèle à cette droite 31 étant au moins égale, sinon égale à quatre fois l'épaisseur Ep, de façon à définir, sur cette portion de face 30, une partie libre 33 d'une largeur au moins égale à Ep, cette partie libre 33 bordant la face 28 de l'autre cavalier 22 qui est la plus éloignée de la face 27 du cavalier 21 située dans le plan d'un bord de la tranche 15, figures 1 et 3.

Toujours selon une réalisation très préférentielle, les moyens pour solidariser la patte de pince 12 avec la tranche 15 de la plaque 10 sont agencés de façon que la portion de face 30 et la face de préhension 13 de la patte de pince 12 soient sensiblement coplanaires, figures 1 et 2.

Toujours selon une autre réalisation très préférentielle, les moyens pour solidariser les deux cavaliers 21, 22 avec la portion de face 30 de la plaque 10 sont agencés de façon que les axes colinéaires des deux orifices 23, 24 des deux cavaliers 21, 22 soient sensiblement situés sur cette portion de face 30, figures 2 et 3.

De façon avantageuse, l'élément selon l'invention pour réaliser une pince vasculaire comporte en outre deux évidements, un premier évidement situé entre les deux cavaliers 21, 22 et un second évidement 40 réalisé dans la partie libre 33 de la portion de plaque 30, ce second évidement bordant la face 28 du cavalier 22 qui est en regard de cette partie libre 33 de la portion de plaque 30, ces premier et second évidements étant réalisés de façon à permettre la rotation des deux cavaliers 21, 22 d'un premier élément monté en coopération avec un second élément identique au premier élément de façon que les axes des quatre orifices 23, 24, 23', 24' des quatre cavaliers soient colinéaires, avec les deux cavaliers 21', 22' du dit second élément, figures 2 et 3.

Selon une autre réalisation très préférentielle, l'élément selon l'invention comporte des encoches 50, figure 2, réalisées sur au moins une face extérieure de la patte de pince 12, c'est-à-dire autre que la face de pincement 13, pour constituer des repères de position d'un anneau élastique 52 dont la fonction sera explicitée ci-après.

La présente invention a aussi pour objet une pince vasculaire qui est constituée par l'assemblage de deux éléments identiques tels que décrits ci-dessus, ces deux éléments étant assemblés, figure 2 et 3, de façon que l'un (en traits continus sur la figure 2) des deux cavaliers 21, 22 d'un des deux éléments soit enfiché entre les deux cavaliers 21', 22' de l'autre élément (en traits interrompus sur cette même figure 2) et que les axes des quatre orifices 23, 24, 23', 24' soient colinéaires, et d'un arbre de rotation 60 d'un diamètre égal au diamètre des quatre orifices, ledit arbre de rotation étant enfiché dans ces quatre orifices.

De plus, cette réalisation permet de prévoir au moins un anneau élastique 52 apte à être situé dans une des encoches 50 en entourant extérieurement les deux pattes de pince 12, 12' des deux éléments assemblés, pour exercer une force de pincement, par exemple sur un vaisseau sanguin, d'une intensité déterminée notamment par les Praticiens.

Dans ce dernier cas, cet anneau élastique 52 présente une forme agencée pour délivrer une tension radiale de contrainte de valeur donnée T qui répond sensiblement à la formule :

$$T = \frac{-2E(R-Ro)}{Ro}$$

dans laquelle E est le module de Young du matériau élastique, et Ro et R sont respectivement les rayons moyens de l'anneau non contraint et contraint.

La description faite ci-dessus, aussi bien de l'élément que de la pince vasculaire est suffisante, avec l'enseignement de l'art antérieur pour comprendre le fonctionnement de la pince vasculaire, fonctionnement qui ne sera donc pas plus amplement décrit ici.

En revanche, il est à remarquer qu'une pince vasculaire réalisée avec deux éléments selon l'invention présente des avantages certains, comme mentionné au préambule de la présente description. Notamment, elle est réalisée avec deux éléments identiques, ce qui réduit le coût de fabrication, notamment par moulage, car ne nécessitant qu'un seul moule. En outre, comme cette pince vasculaire est parfaitement symétrique, quand les pattes 12, 12' enserrent un vaisseau sanguin, par exemple d'une main en cours d'opération, elle n'a pas tendance à vriller et la force de pincement demeure parfaitement appliquée sur le vaisseau sanguin, du fait notamment que les deux surfaces de pincement respectivement des deux éléments sont sensiblement parallèles

## Revendications

1. Elément pour réaliser une pince vasculaire, comportant :

   • une plaque de support (10),
   • une patte de pince (12) comportant une face de préhension (13) et définissant un axe longitudinal,
   • des moyens pour solidariser ladite patte de pince avec la tranche (15) de ladite plaque (10),
   • deux cavaliers (21, 22) sensiblement identiques, chaque cavalier ayant sensiblement la forme d'un secteur de cylindre d'une épaisseur Ep prise selon une parallèle à la génératrice de définition du secteur de cylindre et comportant un orifice traversant (23, 24) ayant un diamètre déterminé et d'axe parallèle à ladite génératrice,

• des moyens pour solidariser les deux cavaliers avec une portion de face (30) de ladite plaque (10) de façon que les axes des deux orifices traversants (23, 24) soient situés sur une même droite (31) perpendiculaire à l'axe longitudinal de ladite patte de pince (12) et que les faces en regard (25, 26) des deux cavaliers (21, 22) soient distantes l'une de l'autre d'une valeur égale à l'épaisseur Ep,

lesdits moyens pour solidariser les deux cavaliers (21, 22) avec la portion de face (30) de ladite plaque (10) étant agencés de façon qu'un (21) des deux cavaliers ait une face (27) située dans le plan d'un bord de la tranche (15) de la plaque (10) perpendiculaire à ladite droite (31), et que ladite portion de face (30) soit sensiblement plane,

**caractérisé par le fait que** la largeur de ladite portion de face (30) prise selon une direction parallèle à ladite droite (31) étant au moins sensiblement égale à quatre fois l'épaisseur Ep, de façon à définir, sur la portion de face (30), une partie libre (33) bordant la face (28) de l'autre cavalier (22) qui est la plus éloignée de la face (27) du cavalier (21) située dans le plan d'un bord de ladite tranche (15).

2.  Elément selon la revendication 1, **caractérisé par le fait que** lesdits moyens pour solidariser ladite patte de pince (12) avec la tranche (15) de ladite plaque (10) sont agencés de façon que ladite portion de face (30) et la face de préhension (13) de la patte de pince (12) soient sensiblement coplanaires.

3.  Elément selon la revendication 2, **caractérisé par le fait que** lesdits moyens pour solidariser les deux cavaliers (21, 22) avec la portion de face (30) de ladite plaque (10) sont agencés de façon que les axes colinéaires des deux orifices (23, 24) des deux cavaliers (21, 22) soient sensiblement situés sur ladite portion de face (30).

4.  Elément selon la revendication 3, **caractérisé par le fait qu'**il comporte deux évidements, un premier évidement situé entre les deux cavaliers (21, 22) et un second évidement (40) réalisé dans ladite partie libre (33) de la portion de plaque (30), ce dit second évidement bordant la face (28) du cavalier (22) qui est en regard de cette partie libre (33) de la portion de plaque (30), ces premier et second évidements étant réalisés de façon à permettre la rotation des deux cavaliers (21, 22) d'un premier élément monté en coopération avec un second élément identique au premier élément de façon que les axes des quatre orifices (23, 24, 23', 24') des quatre cavaliers soient colinéaires, avec les deux cavaliers (21', 22') du dit second élément.

5.  Elément selon l'une quelconque de revendications précédentes, **caractérisé par le fait qu'**il comporte des encoches (50) réalisées sur ladite patte de pince (12) pour constituer des repères de position d'un anneau élastique (52).

6.  Pince vasculaire, **caractérisée par le fait qu'**elle est constituée par l'assemblage de deux éléments identiques définis selon l'une des revendications précédentes, ces deux éléments étant assemblés de façon que l'un des deux cavaliers (21, 22) d'un des deux éléments soit enfiché entre les deux cavaliers (21', 22') de l'autre élément et que les axes des quatre orifices (23, 24, 23', 24') soient colinéaires, et d'un arbre de rotation (60) d'un diamètre égal au diamètre des quatre orifices, ledit arbre de rotation étant enfiché dans ces quatre orifices.

7.  Pince vasculaire selon les revendications 5 et 6, **caractérisée par le fait qu'**elle comporte au moins un anneau élastique (52) entourant extérieurement les deux pattes de pince des deux éléments assemblés, ce dit anneau élastique étant situé dans une des encoches (50).

8.  Pince vasculaire selon la revendication 7, **caractérisée par le fait que** ledit anneau élastique (52) présente une forme agencée pour délivrer une tension radiale de contrainte de valeur donnée T qui répond sensiblement à la formule :

$$T = \frac{-2E(R - Ro)}{Ro}$$

dans laquelle E est le module de Young du matériau élastique, et Ro et R sont respectivement les rayons moyens de l'anneau non contraint et contraint.

**Patentansprüche**

1.  Element zum Herstellen einer Gefäßklemme, umfassend:

    - eine Trägerplatte (10),
    - eine Klemmenklaue (12), die eine Greiffläche (13) aufweist und eine Längsachse definiert,
    - Mittel zum Befestigen der Klemmenklaue an dem Rand (15) der Platte (10),
    - zwei Ösen (21, 22), die im Wesentlichen gleich sind, wobei jede Öse im Wesentlichen die Form eines Zylinderabschnitts mit einer Dicke Ep, die entlang einer Parallelen zu der Definitionslinie des Zylinderbereichs genommen wird, und eine Durchgangsöffnung (23, 24) mit einem vorbestimmten Durchmesser und einer Achse parallel zu der Linie aufweist,

- Mittel zum Befestigen der beiden Ösen an einem Flächenabschnitt (30) der Platte (10), derart, dass die Achsen der beiden Durchgangsöffnungen (23, 24) auf derselben Geraden (31) senkrecht zur Längsachse der Klemmenklaue (12) liegen und dass die einander zugewandten Flächen (25, 26) der beiden Ösen (21, 22) voneinander um einen Wert beabstandet sind, der gleich der Dicke Ep ist,

wobei die Mittel zum Befestigen der beiden Ösen (21, 22) an dem Flächenabschnitt (30) der Platte (10) so angeordnet sind, dass eine (21) der beiden Ösen eine Fläche (27) besitzt, die in der Ebene einer Randkante (15) der Platte (10) senkrecht zu der Geraden (31) liegt, und der Flächenabschnitt (30) im Wesentlichen eben ist, **dadurch gekennzeichnet, dass** die Breite des Flächenabschnitts (30), genommen in einer Richtung parallel zu der Geraden (31), mindestens gleich der vierfachen Dicke Ep ist, derart, dass auf dem Flächenabschnitt (30) ein freier Teil (33) definiert ist, der an die Fläche (28) der anderen Öse (22) angrenzt, die von der Fläche (27) der Öse (21), die in der Ebene einer Randkante (15) liegt, am weitesten entfernt ist.

2. Element nach Anspruch 1, **dadurch gekennzeichnet, dass** die Mittel zum Befestigen der Klemmenklaue (12) an dem Rand (15) der Platte (10) so angeordnet sind, dass der Flächenabschnitt (30) und die Greiffläche (13) der Klemmenklaue (12) im Wesentlichen koplanar sind.

3. Element nach Anspruch 2, **dadurch gekennzeichnet, dass** die Mittel zum Befestigen der beiden Ösen (21, 22) an dem Flächenabschnitt (30) der Platte (10) so angeordnet sind, dass die kolinearen Achsen der beiden Öffnungen (23, 24) der beiden Ösen (21, 22) im Wesentlichen auf dem Flächenabschnitt (30) liegen.

4. Element nach Anspruch 3, **dadurch gekennzeichnet, dass** es zwei Aussparungen aufweist, eine erste Aussparung, die sich zwischen den beiden Ösen (21, 22) befindet, und eine zweite Aussparung (40), die in dem freien Teil (33) des Plattenabschnitts (30) ausgebildet ist, wobei diese zweite Aussparung an die Fläche (28) der diesem freien Teil (33) des Plattenabschnitts (30) zugewandten Öse (22) angrenzt, und diese erste und diese zweite Aussparung derart ausgebildet sind, dass eine Drehung der beiden Ösen (21, 22) eines ersten Elements ermöglicht wird, das zusammenwirkend mit einem zweiten Element, das mit dem ersten Element identisch ist, so montiert ist, dass die Achsen der vier Öffnungen (23, 24, 23', 24') der vier Ösen kolinear sind mit den beiden Ösen (21', 22') des zweiten Elements.

5. Element nach einem der vorhergehenden Ansprüche, **dadurch gekennzeichnet, dass** es Kerben (50) aufweist, die in der Klemmenklaue (12) ausgebildet sind, um Positionsmarkierungen eines elastischen Rings (52) zu bilden.

6. Gefäßklemme, **dadurch gekennzeichnet, dass** sie gebildet ist durch den Zusammenbau zweier gleicher Elemente nach einem der vorhergehenden Ansprüche, wobei diese beiden Elemente so zusammengefügt sind, dass eine der beiden Ösen (21, 22) eines der beiden Elemente zwischen die beiden Ösen (21', 22') des anderen Elements gesteckt ist und dass die Achsen der vier Öffnungen (23, 24, 23', 24') kolinear sind, und durch eine Drehwelle (60), deren Durchmesser gleich dem Durchmesser der vier Öffnungen ist, wobei die Drehwelle in diese vier Öffnungen gesteckt ist.

7. Gefäßklemme nach den Ansprüchen 5 und 6, **dadurch gekennzeichnet, dass** sie mindestens einen elastischen Ring (52) umfasst, der die beiden Klemmenklauen der beiden zusammengefügten Elemente außen umgibt, wobei dieser elastische Ring in einer der Kerben (50) angeordnet ist.

8. Gefäßklemme nach Anspruch 7, **dadurch gekennzeichnet, dass** der elastische Ring (52) eine Form aufweist, die so beschaffen ist, dass er eine radiale Beanspruchungsspannung mit einem vorbestimmten Wert T bereitstellt, der im Wesentlichen die Formel

$$T = \frac{-2E\left(R - R_0\right)}{R_0}$$

erfüllt, wobei E der Elastizitätsmodul des elastischen Materials ist und Ro und R entsprechend die mittleren Radien des nicht belasteten Rings bzw. des belasteten Rings sind.

**Claims**

1. An element for producing a vascular clip, comprising:

   • a support plate (10),
   • a clip tab (12) with a gripping face (13) and defining a longitudinal axis,

• means for securing said clip tab and the edge face (15) of said plate (10),
• two substantially identical brackets (21, 22), each substantially having the shape of a cylinder sector with thickness Ep taken along a line parallel to the generatrix defining the cylinder sector, with a through hole (23, 24) having a definite diameter and an axis parallel to said generatrix,
• means for securing the two brackets and a face portion (30) of said plate (10) in such a way that the axes of the two through holes (23, 24) are located along the same straight line (31) perpendicular to the longitudinal axis of said clip tab (12) and the opposite faces (25, 26) of the two brackets (21, 22) are set apart from each other by a value equal to the thickness Ep, said means for securing the two brackets (21, 22) and the face portion (30) of said plate (10) being arranged in such a way that one (21) of the two brackets has a face (27) located on the plane of an edge of the edge face (15) of the plate (10) perpendicular to said straight line (31), and said face portion (30) is substantially flat, **characterized in that** the width of the said face portion (30) taken along a direction parallel to said straight line (31) is at least substantially equal to four times the thickness Ep so as to define, on the face portion (30), a free part (33) bordering the face (28) of the other bracket (22) that is the farthest away from the face (27) of the bracket (21) located on the plane of an edge of said edge face (15).

2. An element according to claim 1, **characterized by** the fact that said means for securing the clip tab (12) and the edge face (15) of the plate (10) are arranged in such a way that the face portion (30) and the gripping face (13) of the clip tab (12) are substantially coplanar.

3. An element according to claim 2, **characterized by** the fact that said means for securing the two brackets (21, 22) and the face portion (30) of said plate (10) are arranged in such a way that the collinear axes of the two holes (23, 24) of the two brackets (21, 22) are substantially located on said face portion (30).

4. An element according to claim 3, **characterized by** the fact that it comprises two recesses, the first of which is located between the two brackets (21, 22) and the second (40) is made in the free part (33) of the plate portion (30), the second recess bordering the face (28) of the bracket (22) that is opposite the free part (33) of the plate portion (30), and the first and second recesses are made in such a way as to allow the rotation of the two brackets (21, 22) of a first element mounted in engagement with a second element identical to the first one in such a way that the axes of the four holes (23, 24, 23', 24') of the four brackets are collinear, with the two brackets (21' 22') of the second element.

5. An element according to any of the foregoing claims **characterized by** the fact that it comprises notches (50) made on said clip tab (12) to make position references for an elastic ring (52).

6. A vascular clip **characterized by** the fact that it is made up of the assembly of two identical elements such as those defined in any of the foregoing claims, the two elements being assembled in such a way that one of the two brackets (21, 22) of one of the two elements is fitted between the two brackets (21', 22') of the other element and that the axes of the four holes (23, 24, 23', 24') are collinear, and of a rotation pin (60) with a diameter equal to the diameter of the four holes, which rotation pin is fitted in the four holes.

7. A vascular clip according to claims 5 and 6, **characterized by** the fact that it comprises at least one elastic ring (52) that externally surrounds the two clip tabs of the two assembled elements, said elastic ring being located in one of the notches (50).

8. A vascular clip according to claim 7, **characterized by** the fact that said elastic ring (52) has a shape designed to apply radial tensile force in given value T that is substantially based on the formula:

$$T = \frac{-2E(R - Ro)}{Ro}$$

wherein E is the Young's modulus of the elastic material, and Ro and R are the average radii of the ring when unstrained and strained, respectively.

*Fig. 1*

*Fig. 3*

*Fig. 2*

**EP 3 651 661 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5571125 A
- EP 0105414 A
- US 5103839 A
- WO 2004073487 A